# EUROPEAN PATENT APPLICATION

(11) **EP 1 978 372 A2**
(43) Date of publication of application: **08.10.2008**
(21) Application number: 08160660.0
(22) Date of filing: 29.09.2006
(51) Int. Cl.: G01R 33/38

(54) **MRI apparatus and MRI method using such apparatus**

(62) Divisional of application: 06121574.5
(71) Applicant: Esaote S.p.A., 20100 Milano (IT)
(72) Inventor: Satragno, Luigi, I-16122, Genova (IT); Biglieri, Eugenio, I-15024, Masio (IT)
(74) Representative: Karaghiosoff, Giorgio Alessandro

(57) **Abstract**

A MRI apparatus comprising a magnet structure which delimits a cavity in or through which a body under examination or a part thereof is received, and which includes means for generating a magnetic field in said cavity, as well as means for causing the body under examination or the part thereof to emit nuclear magnetic resonance signals and means for receiving said nuclear magnetic resonance signals. In a first aspect of the invention, the magnet structure delimits a cavity whose shape provides at least one access opening through which patients can access said cavity by simply walking therein.

The invention includes means for positioning and supporting a patient in several different postures within the patient receiving space and particular signal receiving means and patient stressing means suitable for the apparatus with the above features.

## Description

This invention relates to a MRI apparatus comprising a magnet structure which delimits a cavity in or through which a body under examination or a part thereof is received, and which includes means for generating a magnetic field in said cavity, as well as means for causing the body under examination or the part thereof to emit nuclear magnetic resonance signals and means for receiving said nuclear magnetic resonance signals.

MRI apparatus typically comprise a magnet structure, which structure is composed of a plurality of wall elements and/or columns or other load bearing elements, which are connected together to delimit a cavity for receiving the body of a patient or at least a part thereof, and at least one or a plurality of openings for said body and/or part thereof to access said cavity. Furthermore, the magnet structure also has means for generating a magnetic field permeating the cavity or a portion of the whole volume of said cavity, which means may include permanent magnets, resistive magnets or superconducting magnets or combinations of such magnets. These magnetic field generating means are supported and/or wholly or partly form the wall elements or the construction elements of the magnet structure.

In addition to the above magnet structure, having the function to delimit the cavity and generating the magnetic field, the MRI apparatus generally comprises so-called gradient coils for generating variable magnetic fields with selection and encoding functions, allowing to reconstruct the spatial relationship between resonance signals and the topological position of the source. Finally, required components include the transmitting coil, whereby a RF exciting pulse is generated, and the receiving coil which picks up the RF magnetic resonance signals emitted from the body under examination or the part thereof being imaged. Further auxiliary equipment or devices and/or units are intended as prior art, which help to improve or control parameters such as electromagnetic and magnetic noise, thermal drifts, electronic drifts, etc.

In the specific field of MR imaging, which is a widely used term in the art to define Magnetic Resonance imaging, tow different families of apparatus are known, i.e. Total Body and Dedicated apparatus. The former have large magnet structures that generate large magnetic fields and their patient receiving cavities, as well as the accesses to such cavities have such a size as to allow the whole body to be received therein. Conversely, dedicated apparatus have magnet structures adapted to generate relatively low-strength magnetic fields, but both the structure and the cavity have a small size, so that only limited anatomical regions or limbs may be imaged therein, such as a lower or upper limb, a shoulder and/or a head.

Magnetic resonance imaging is increasingly used in medicine for orthopedic diagnostics. A critical factor in orthopedic diseases, in addition to the morphological condition of bone tissues, is functional dynamic behavior, especially in different stress conditions.

Therefore, it is important for the patient to be allowed to assume several different postures and be subjected to mechanical stresses as close as possible to natural conditions.

In certain prior art apparatus, various kinds of examinations may be performed with the patient in various positions and stress conditions, which mostly only simulate natural postures and stresses. In these apparatus, the devices for allowing the patient to assume the different postures and the best position in the cavity, as well as the devices required for resonance signal acquisition are different in terms of appearance and structure from the devices used by patients in their daily life, and generate psychological stress, fear and anxiety, causing muscular contractions which may at worst affect natural postures and generate restlessness conditions in the patient, thereby causing difficulties for the latter to keep still for the required examination time.

The invention mainly addresses the problem of providing a MRI apparatus that allows easy examination of several different anatomic regions under natural load or stress conditions, without requiring highly technically complex and expensive accessories and/or constructions and without inducing psychological stress, i.e. anxiety or fear, in patients.

Therefore, the invention solves the above problem by providing a MRI apparatus as described hereinbefore, wherein said magnet structure delimits a cavity whose shape provides at least one access opening through which patients can access said cavity by simply walking therein.

In a further aspect of the invention, as described above, the apparatus includes means for positioning and/or bearing and/or retaining and/or supporting a patient or for assisting placement thereof, which are mountable and removable and/or displaceable in and/or out of the patient receiving space separately or in combination.

Thanks to the above feature, the patient can assume various postures, using very simple means, which give the patent the highest autonomy and freedom of movement, which postures are useful for performing an examination, particularly with an anatomic region, or the like, under physiological stress conditions.

The means for positioning and/or bearing and/or retaining and/or supporting a patient or for assisting placement thereof are very simple and inexpensive. As a rule, these means are commonly used in daily life, wherefore they have a simple construction and are user-friendly. Using simple, low-cost means, which cause no fear or stress to patients, a high versatility may be achieved in terms of the variety of possible patient postures. The means for positioning and/or bearing and/or retaining and/or supporting a patient may be also provided in combinations. The patient is always free to move and autonomously assume the postures required for the examination without having its freedom of movement restricted and without being subjected to forced control by the medical staff, therefore without having the sensation of being in serious pathological conditions, or that his/her conditions are more serious than they actually are.

The means for positioning or retaining a patient may also easily act as guides in or through which several different devices or means may be received, such as resonance signal receiving means and/or cables for connection of the output of said means to an electronic processing unit, which is contained in the apparatus associated to the magnet structure and, for example, is located outside the latter.

As described in further detail hereafter, and claimed in the dependent claims, several different types of such means for positioning and/or bearing and/or retaining and/or supporting the patient or for assisting placement thereof may be provided. Without limitation, the patient receiving space may contain chairs, armchairs, patient tables, convertible armchairs, or other kinds of seats. There may be further provided handles or gripping, supporting and/or retaining means which allow the patient to assume intermediate positions, such as prone or backwardly arched positions or intermediate bent knee positions or other positions.

All the above means for positioning and/or bearing and/or retaining and/or supporting a patient or for assisting placement thereof may be position-adjustable relative to the magnet structure and the imaging volume and/or to other means for positioning and/or bearing and/or retaining and/o supporting a patient or for assisting placement thereof, which are provided in combination therewith.

In another improvement of the invention, arrangements are provided for bringing the receiving coil as close as possible to the relevant anatomic region, without restricting the patient's freedom of movement and adding weight to be carried by the patient.

As a rule, receiving coils are or may be permanently coupled to the means for positioning and/or bearing and/or retaining and/or supporting a patient or for assisting placement thereof as defined above, or are integrated in the magnet structure together with the other devices of the apparatus.

Besides providing a magnet structure having a patient receiving space with access openings of such a size as to allow the patient to enter and exit by simply walking into and out of said patient receiving space through said openings, the invention advantageously provides resonance signal receiving means that can be removably attached directly to the patient body, at one or more separate anatomic regions to be imaged.

A number of embodiments of such receiving means, particularly in the form of receiving coils, will be described below and claimed in the dependent claims. These embodiments provide arrangements for adapting wearable receiving coils to a certain range of patient body sizes without affecting signal quality, signal power and/or signal-to-noise ratio, as well as without causing pain to patients or restricting conditions which might generate psychological stress or anxiety.

By placing the receiving coil closer to the signal source, a better signal-to-noise ratio is obtained, or at least the quality standards for such ratio may be kept constant in spite of a limitation of the static field strength and of excitation pulses. The shorter distance between the receiving coil and the relevant anatomic region compensates for the weaker resonance signals emitted by a relatively weak magnetic field. Therefore, using the construction parameters of total body magnets, the signal may be improved.

Still regarding the freedom of movement of patients, even when subjected to use or asked to make use of auxiliary means for assuming the required posture, in order to control stress, for instance on the spine, the invention provides means for adjusting gravity stress on the body under examination.

These means are, for instance a garment-like and/or wearable element containing one or more ballast elements of predetermined weight that may be permanently and/or removably held therein and/or replaced with other ballast elements of different weight.

According to an advantageous embodiment, the garment-like and/or wearable element for supporting the ballast means is the same garment-like or wearable element that is used for supporting the antenna or the receiving coil and/or any other resonance signal receiving means or imaging means.

These means are also very simple and cost-effective, of simple construction and user-friendly both for the operating staff and the patient under examination. The stress imparted to the patient is comparable to ordinary daily life activity and allows to perform functional MRI examinations on specific limbs under motion and stress conditions as close as possible as those that are normally experienced by the patient.

As mentioned above, the invention also addresses a method and, regarding such method for performing MRI examinations, it has the purpose of providing a manner of performing such examinations which reduces psychological stress on the patient by avoiding any access or imaging arrangements that might cause the patient to feel he/she is in a serious pathological condition, possibly more serious than it actually is. Also, the method should allow examination of as many anatomic regions as possible and as many patient postures as possible for at least some of such anatomic regions.

A MRI method using a MRI apparatus according to the invention involves the use of a magnet structure which delimits a cavity in or through which a body under examination or a part thereof is received, and which includes means for generating a magnetic field in said cavity, as well as means for causing the body under examination or the part thereof to emit nuclear magnetic resonance signals and means for receiving said nuclear magnetic resonance signals. In the method of this invention, the cavity has a shape that provides at least one access opening through which patients can access said cavity by simply walking therein, and said method comprises the steps of:
- having the patient access the imaging cavity by autonomous walking,
- positioning the patient by having him/her move autonomously as instructed by the operating personnel;
- performing MR imaging.

It will be appreciated that the invention always allows patient to access the apparatus by autonomous walking or other autonomous action even using auxiliary locomotion means, such as a manually or motor driven wheelchair. The apparatus of the invention further allows patient access using means pushed by third parties, such as a patient table, a convertible armchair or the like, e.g. as disclosed in patent EP 913,122 or US 6,346,814.

It may be further envisaged that the patient accesses the patient receiving space by simply walking therein and that a patient table, an armchair, a chair or another element for supporting the patient in a predetermined posture and/or a predetermined position relative to the magnetic structure is introduced or present therein, and the patient autonomously sits or lies thereon. The patient table, armchair, chair or any other support element may be arranged to move on wheels or skids and/or to be locked in one or more predetermined positions within the patient receiving space of the magnet structure thanks to means for stopping motion in the proper position, such as adjustable stops or abutments and/or means for indication of proper positioning allowing to stop the motion of the above patient supporting means.

The following detailed description and the dependent claims describe certain particular embodiments of the present method.

As mentioned above, the dependent claims relate to improvements of this invention.

The characteristics of the invention and the advantages derived therefrom will appear more clearly from the following description of a few non limiting embodiments, illustrated in the annexed drawings, in which:
Fig. 1 is a perspective view of an exemplary magnet structure according to this invention.
Fig. 2 is a view showing the channel between the two opposite pole pieces of the magnet structure in the direction of the longitudinal axis, which channel forms the patient receiving space and in which the patient is shown in a possible examination position.
Figs. 3 to 7 are sectional views taken along a vertical center plane parallel to the surfaces of the pole pieces of the magnet structure as shown in the previous figures and, in each of which Figures 3 to 7 the patient is shown in various postures, obtained thanks to one or more means for positioning and/or bearing and/or retaining and/or supporting the patient or for assisting placement thereof.
Fig. 8 is a diagrammatic view of an exemplary wearable coil, in the form of a band to be fastened around a part of the patient body.
Fig. 9 is a diagrammatic view of an exemplary construction of a receiving coil having flexible and extensible conductors, which are supported by a garment by means for removable attachment of conductors to such garment.
Figs. 10 and 11 are schematic views of different exemplary garment-conductor combinations, using means for removable attachment of conductors.
Figure 11 shows, like Figure 2, a patient standing in the patient receiving space of the magnet structure of the present apparatus, which patient wears a gravity ballast garment.

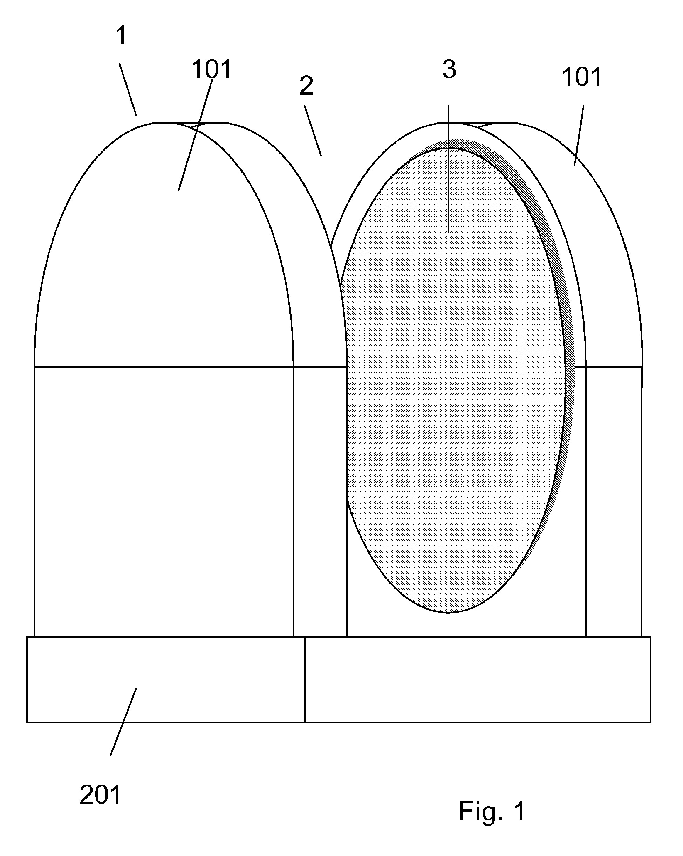

Particularly referring to Figure 1, a MRI apparatus generally comprises a magnet structure 1 which delimits a cavity 2 in or through which a body under examination or a part thereof P is received, and which includes means 3 for generating a magnetic field in said cavity, as well as means for causing the body under examination or the part thereof P to emit nuclear magnetic resonance signals and means for receiving said nuclear magnetic resonance signals.

The construction of magnetic resonance imaging apparatus has been long known. The magnet structure has the function of delimiting a space for receiving a patient or a part of the patient's body and of generating a magnetic field which permeates at least partly the volume of the patient receiving space. The magnet structure is also associated to so-called gradient coils for generating time dependent magnetic fields as well as coils for transmitting matter excitation pulses, which are generally radio frequency pulses, as well as at least one coil for receiving the radio frequency signals resulting from matter response to the excitation pulses.

A general introduction to the principles of operation of MRI apparatus is contained in: "Quaderni di RM, Basi Fisiche della Risonanza Magnetica" A. Desgrez, J.Bittoun, I.Idy-Peretti, Edizione Franco Milani 1992 Masson.

The means for generating the static field that permeates at least partly the patient receiving space may include permanent magnets and/or resistive magnets and/or superconducting magnets.

The type of field generating means has a minor incidence upon the operation principle of MRI apparatus. Construction variants are mainly associated to the means for controlling the resistive or superconducting magnets which require power in coils combined with cores and the corresponding drivers and power supplies. Temperature control is generally always provided even in permanent magnets, however in superconducting magnets in addition to reducing or compensating for thermal drifts of magnetic fields, this control also allows to maintain the conditions in which the coils have a superconducting behavior.

The MRI apparatus has many more components in addition to the magnet structure and the above mentioned operating units, however these components are known parts, which are not related to the present concepts.

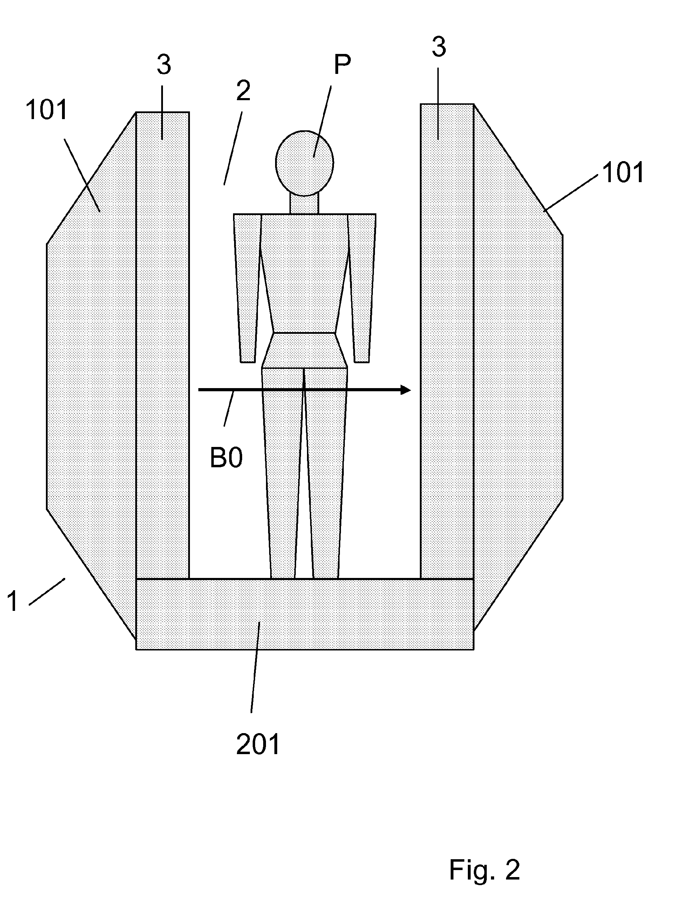

As shown in Figures 1 and 2, the magnet structure has two opposite pole pieces 101 delimiting a cavity whose shape provides at least one access opening through which patients can access said cavity by simply walking therein. Particularly, in this example, the cavity has the shape of a passage for connection of two opposite openings on two opposite vertical sides. The latter may also be parallel or substantially parallel to each other and to the direction of the magnetic field between the pole pieces 101.

Regarding the size, the cavity and the at least one access opening have such a width as to allow the patient to access it by walking and/or remain in such cavity in a position in which the axis that joins his/her shoulders is parallel to the width of the cavity.

In particular conditions, the cavity and the at least one access opening, particularly the two opposite access openings have such a width as to allow access by a wheelchair or similar device, either driven by the person sitting thereon or pushed by third parties.

The patient may be also introduced in the cavity 2 using a patient table, a convertible armchair and/or an armchair or the like, instead of the wheelchair, after laying the patient thereon outside the magnet structure. The patient table, armchair and/or other similar devices may also have means for adjusting the height and/or tilt of the patient support plane about one or more axes so that the patient may be tilted with different orientations in space.

The two opposite field generating pole pieces form or are supported by two opposite vertical walls which are spaced to such an extent as to form the above mentioned patient receiving space and at least one access opening at least at one end side. The means for excitation of the body under examination or a part thereof for emission of resonance signals (not shown in detail and generally consisting of at least one transmitting coil) are integrated in or supported by said walls or are combined with said pole pieces 101.

In this example, the wall that connects the two pole pieces 101 and/or the walls supporting such two pole pieces 101 is the support base for the magnet structure and the walking surface for the patient when he/she is within the patient receiving space.

A number of variants of the magnet structure may be provided, such as different rotations of the structure as shown in Figures 1 and 2, in which the wall 201 is in an upright position, wherefore the magnet structure is composed of three closed vertical walls, formed by the pole pieces 101 or the associated support walls, and the wall 201, and the walking surface is the floor itself or another base. It may be also envisaged that the wall 201 form the ceiling wall, with the structure of Figures 1 and 2 being turned 180°. In lieu of a C- or U-shaped magnet as shown herein, there may be provided magnet structures having a closed annular structure and one or two open sides transverse to the axis of the annular structure, in which magnets the two pole pieces 101 have a horizontal or substantially horizontal orientation and are held at a distance from each other by two or more columns arranged along the periphery of said pole pieces.

The base 201 that, in the specific non-limiting example of the figures, acts as a connection wall between the two opposite walls that form or support the pole pieces 101 of the magnet structure, may have one, two or more steps on the opening side, for access to the imaging space. Otherwise, to allow access by disabled people and/or by carriage-mounted means of transport, such as patient tables, armchairs or the like, there are provided at least two parallel climbing ramps at the at least one access opening, along parallel strips disposed at the same distance from each other as the wheels of a wheelchair, a patient table, an armchair, or the like, or one climbing ramp whose width corresponds to the total width of the base.

A balustrade and/or lateral gripping and retainer means may be further provided at least in the area of the steps and/or ramps and at least on one side or on both sides thereof, for assisting the patient in sliding into the space 2.

While Figure 2 shows a standing patient, with the axis that joins his/her two shoulders substantially perpendicular to the pole pieces and/or parallel to the magnetic field therebetween, the patient may willingly take any position or posture in the imaging space. For assisting the patient in assuming certain postures, which pose particular problems in terms of balance and/or physical effort, considering that imaging sequences of a MRI apparatus may be relatively long, the MRI apparatus of the invention also comprises means for positioning and/or bearing and/or retaining and/or supporting the patient or for assisting placement thereof. These are advantageously mountable, removable or even displaceable, separately or together, to and from the imaging space, thanks to their possibly motor-driven carriage-mounted design. Any type of particular and specific positioning and/or bearing and/or retaining and/or supporting means may be used, provided that the patient is not forced to assume any position, and that he/she can assume it by a wholly voluntary action, and preferably by a natural action or movement. This description only mentions a few examples, which are to be intended without limitation, although they cover the most typical and/or useful patient postures, especially for morphological and functional imaging.

Furthermore, while Figure 2 shows the standing patient P with the axis that joins his/her shoulders parallel or substantially parallel to the static field B0 generated between the pole pieces, the patient may assume any position with any inclination of the axis that joins his/her shoulders with respect to the magnetic field direction. Also, different orientations may be provided for different body parts, thence the patient may assume a posture in which the shoulders and the pelvis have different orientations, by rotating his/her torso, or other similar postures.

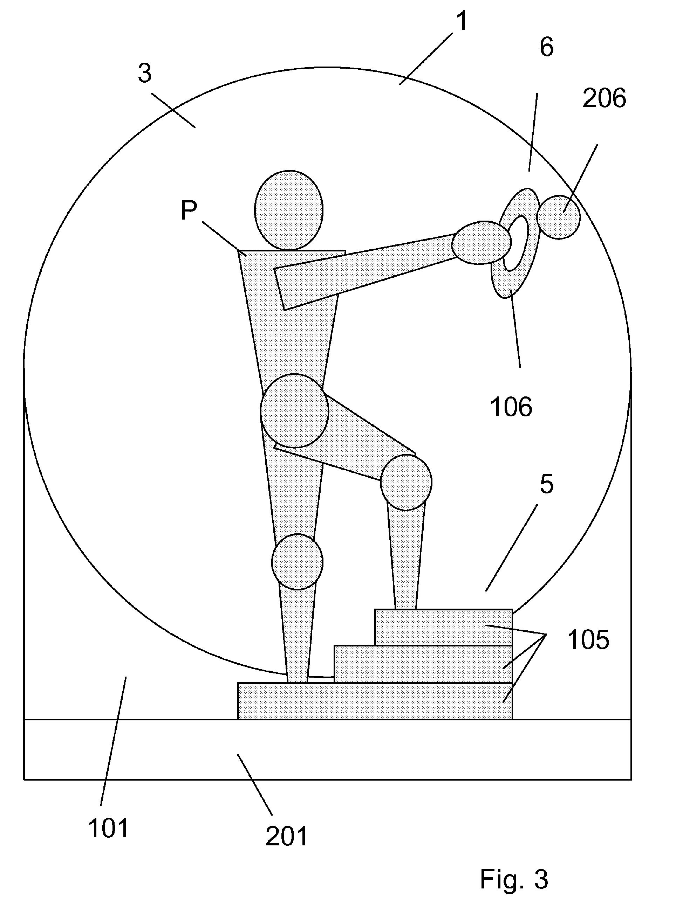

In a first example, as shown in Figure 3, the positioning means consist of one or more platforms 5 having different heights and an access side composed of one or more successive steps. These platforms 5 may be removably mounted in several different positions to the base 201 or the walking surface of the magnet structure within the patient receiving space. The platforms may also have a carriage-like support base, allowing displacement within the patient receiving space 2, removal therefrom and placement in a predetermined position. Braking means and/or stopping means, such as abutments, removable and/or adjustable limit stops, may allow the platform 5 to be temporarily locked within the patient receiving space 5 in one or more predetermined positions corresponding to one or more predetermined anatomic region- and/or organ-specific imaging positions. The static field B0 normally exhibits optimal homogeneity in a space volume extending over a portion of the total volume of the patient receiving space 2, which is generally approximately centered with respect to such total volume. Therefore, diagnostic imaging of an anatomic region or organ requires such region or organ to be placed in the partial volume in which the best magnetic field homogeneity can be achieved. This partial volume is known in the art as imaging volume.

According to an improvement, the platform has a modular construction, i.e. is composed of multiple platform elements 105, each having a predetermined height, e.g. a minimum unit height. Also, one or more platform elements may be diminished or increased in height, allowing height adjustment in a range from a minimum value to a maximum value. By this arrangement, coarse patient position adjustment with respect to the imaging volume, for the above purpose of centering the organ and/or anatomic region with the imaging volume, is performed using the modular elements, whereas fine position adjustment occurs thanks to the height or thickness adjustable element. In a variant thereof, this element is provided as a common base element which has additional carriage characteristics, so that only one module of the set is adjustable in thickness or height, whereas all the others are stationary, hence simpler and less expensive.

The platform elements 105 may be mounted in superimposed arrangements for making platforms 5 of various heights. They may be also arranged in laterally staggered position with respect to vertical alignment, to form a stepped access side.

A further example of the means for positioning and/or bearing and/or retaining and/or supporting the patient or for assisting placement thereof, that may be provided separately or in combination with other positioning means consists of one or more gripping handles 6 or other gripping means, which may be either permanently or removably fixed in several different positions with respect to the walls that form and/or support the pole pieces 101 and/or with respect to the base 201 and/or with respect to the platform 5 and to other means for positioning and/or bearing and/or retaining and/or supporting the patient or for assisting placement thereof, such as those that will be described in the following embodiments.

In Figure 3, the handles 6 are provided in combination with the platform 5 in such a position as to allow the patient to hang thereto with little effort in a position in which the knee and/or other musculoskeletal regions or other articulations are under stress, such as climbing a step. The handles 6 or any other different gripping means are permanently mounted to the magnet structure. Possibly, at least some of the gripping means, combined together, are arranged in multiple fixed positions, whereas at least some of the rest of such handles or gripping means are or may be mounted removably and/or adjustably in terms of position and orientation, and may assume several different positions with respect to the extension of the walls that support or form the pole pieces and/or the base and/or the platform/s.

In Figure 3, the handle 6 comprises a gripping element 106 which is fixed to the magnet structure by means of an upper horizontal beam 206. The gripping element may be secured to the beam by means of skids, which slide along the axis of the beam 206 and may be locked in position. Furthermore, the gripping element may be connected to the skids (not shown) by means of tilt or extension joints. Alternatively or in addition, the walls 101 of the magnet structure may have adjustable supports in one or more predetermined end positions of the beam 206 or two or more different fixed supports in different positions.

A great number of variants for fixation of the gripping means 106 may be envisaged, including any type of brackets or support elements, such as rods, posts and/or beams, each of which may be removably fixed in one or more predetermined positions to the base and/or the walls or the pole pieces of the magnet structure and/or the platform/s or the modular platform elements.

19. An apparatus as claimed in claim 17 or 18, characterized in that the elements for supporting the gripping means and/or the handles are formed by multiple elements, that can be removably coupled together and/or extended or shortened or anyway have an adjustable size.

Referring to Figure, the gripping or retaining means 6 consist of at least one handle, preferably a pair of laterally aligned or misaligned handles 6, which are spaced in such a position as to provide support for forward bending of the standing patient body P, i.e. in a prone position thereof. In this case, the gripping element/s 106 of each handle are mounted to a vertical post 206. This post may be provided in several different lengths or be continuously adjustable in length.

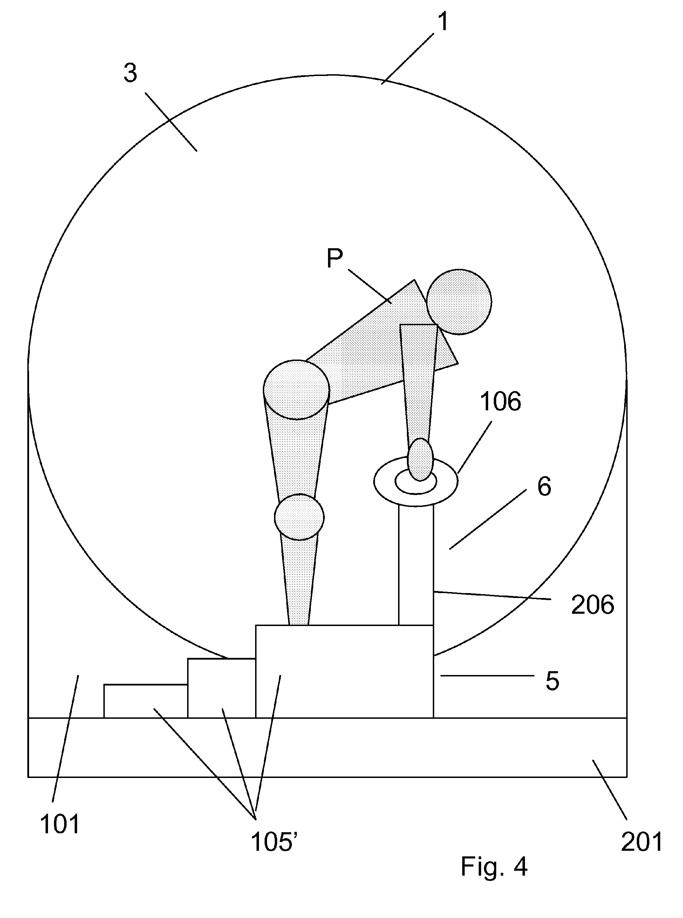

As shown in Figure 4, the handle 6 is provided in combination with a stepped base 5, which is composed of modules 105' having a different design from those described above, as an example of the many different alternative constructions that may be provided therefor. The post/s 206 for the handles 6 may be fixed to one of such modules 105' of the base, preferably in a removable manner.

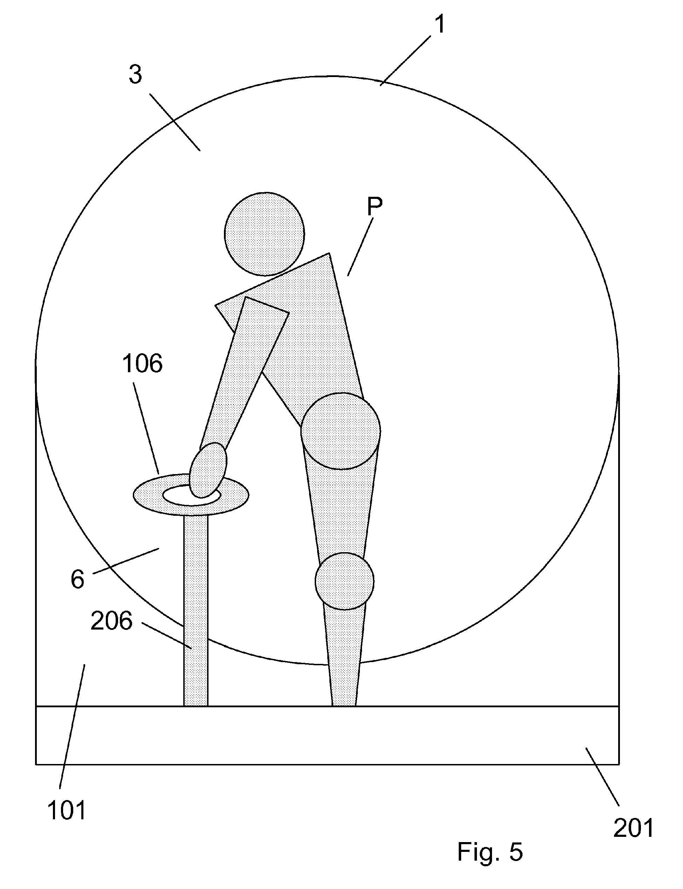

Figure 5 shows a variant handle positioning embodiment, providing at least one handle, preferably a pair of laterally aligned or misaligned handles, which are spaced in such positions as to act as a support for the patient. In the specific condition of the figure, the patient is in a backwardly arched position, said handles being adjusted and positioned to act as a support for backward arching of the patient body. Nevertheless, these handles may be also used for forward arching when suitably adjusted and positioned in the patient receiving space 2.

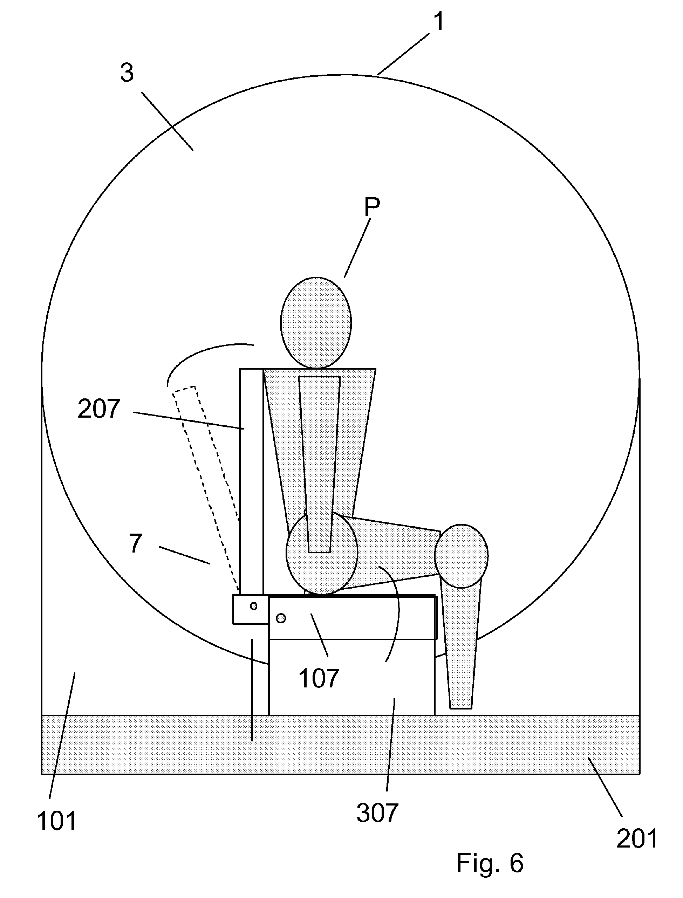

Figure 6 shows a further variant of the means for positioning and/or bearing and/or retaining and/or supporting the patient or for assisting placement thereof, which consist of at least one seat element 7 that may be removably mounted or positioned in different positions within the imaging space and on the base thereof.

The seat element may be provided in combination with one or more of the means 5 or 6 for positioning and/or bearing and/or retaining and/or supporting the patient or for assisting placement thereof may be provided as described above. Like the bases 5, the base 307 of the seat element may have a carriage-mounted design and/or be provided with means for adjusting the seat height position and/or means for tilt adjustment along one or more different axes of the seating surface 107 and/or the backrest 207. The seat 7 has at least one seating surface part 107 which is allowed to tilt along at least one axis, preferably along two perpendicular axes, and especially along at least one transverse axis, i.e. oriented in the seat width and/or magnetic field B0 direction (see Fig. 2). The seat 7 has at least one backrest part 207 which is allowed to tilt along at least one axis, preferably along two perpendicular axes, and especially along at least one transverse axis, i.e. oriented in the backrest width and/or magnetic field B0 direction (see Fig. 2).

The seat 7 may be also provided in combination with footrest elements (not shown) that may be removable and/or adjustable in tilt along at least one transverse axis, i.e. oriented in the backrest width direction, and/or along two perpendicular axes and/or possibly additionally or alternatively adjustable in height.

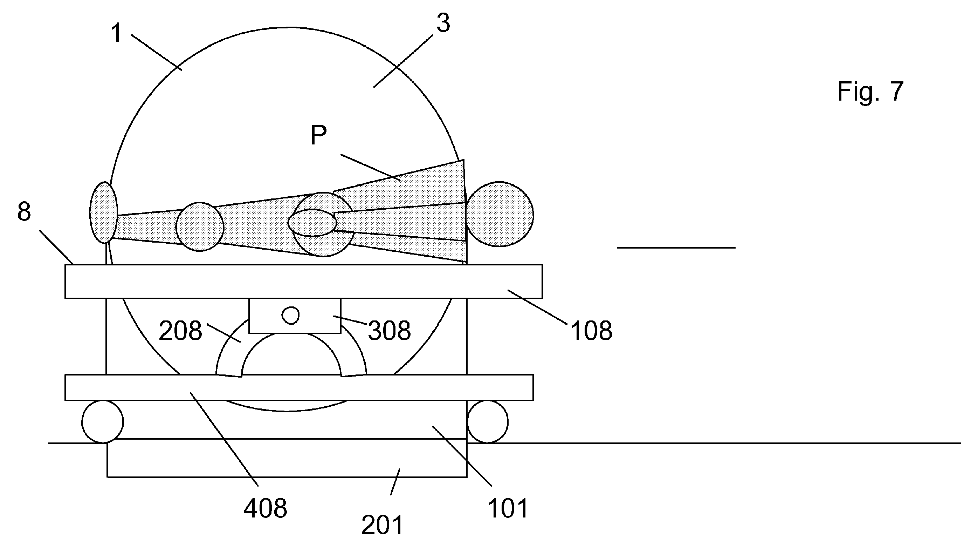

Referring to Figure 7, another positioning means may be a patient table 8. In its simplest form, the patient table 8 may be permanently or removably placed in the patient receiving space 2 and the patient accesses such space by simply walking therein and lies on such patient table 8. The patient table may have height adjustment means in the base and/or the support legs thereof. Several different constructions of patient table means or structures are known and envisageable, having a lifting or height adjustable support top. Furthermore, the patient table and particularly its support top 108 may have means for tilt adjustment of such top 108 along two longitudinal and/or transverse axes or along a single axis or by means of ball joints. Figure 7 shows a schematic example of a tilting support for the plate 108 of the patient table 8. The base part has an arched bracket 208 which is jointed at its top to a lower extension 308 of each lateral longitudinal edge of the patient table top 108 for the latter to be able to tilt about a horizontal axis. Removable locking means release the top so that it is free to tilt to a predetermined position, such removable locking means being then engaged to hold the patient table in the selected tilted position. The removable locking means may be of any type, such as transverse pins to be fitted in coincident holes in one of the extensions 308 of the bracket 208, the bracket having a succession of spaced holes formed therein coincident with the path of the hole of the lower extension 308 as the table top 108 is tilted.

In accordance with a further improvement or alternative arrangement, the patient table 8 may have a carriage-mounted base like the one designated by numeral 408 in Figure 7. In this case, the carriage-mounted base 408 may also have braking means for position locking.

The patient may access the patient receiving space by simply walking therein and only lie on the table when he/she is in such patient receiving space, or he/she may be placed on the table outside the patient receiving space 2 of the magnet structure and be carried into said space on the table 8.

Any other device, such as an armchair and/or a convertible armchair may be provided instead of the patient table.

Besides allowing the patient to assume various postures, the means for positioning and/or bearing and/or retaining and/or supporting the patient or for assisting placement thereof act at least partly as guides or cases for receiving several different devices or means, such as the resonance signal receiving means and the cables for connecting the outputs of such means to an electronic processor unit of the apparatus, associated to the magnet structure and located outside the latter.

Regarding the resonance signal receiving means, typically receiving coils, the invention proposes two solutions, which many be used separately or in combination. One of such solutions consists in integrating the receiving coils and/or the resonance signal receiving means in the walls 101 that form or support the pole pieces. The receiving coils may be held in such walls or supported thereby and be integrated in the pole pieces.

According to the second solution, the resonance signal receiving means may be removably fixed directly to the body under examination or a part thereof. These resonance signal receiving means, i.e. receiving coils are removably fixable directly to the patient body in the area of one or more separate anatomic regions to be imaged.

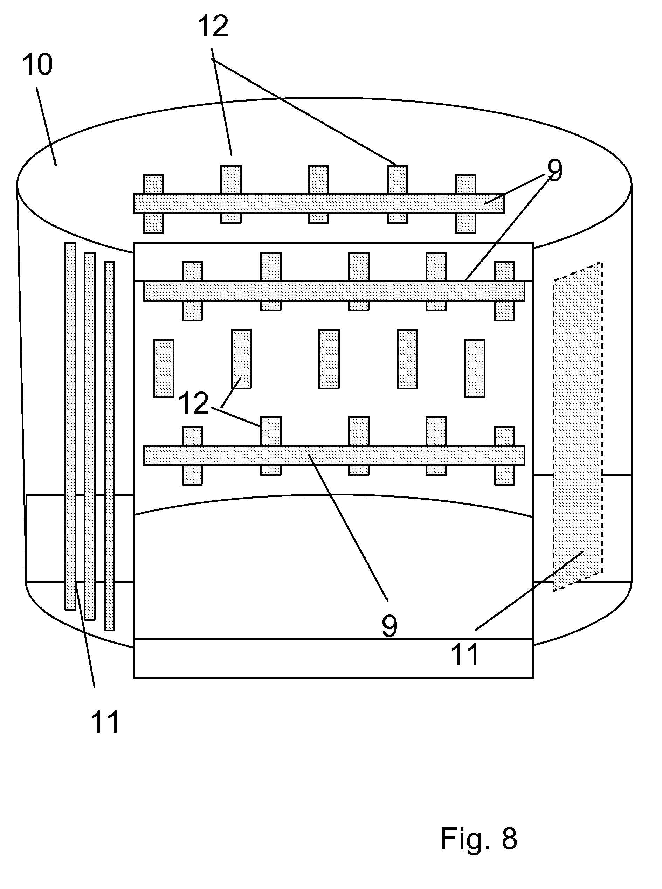

According to an advantageous feature of this invention, these receiving means comprise an electromagnetic (RF) signal receiving antenna, composed of one or more conductors 9, which are permanently or removably fitted in or on a garment-like and/or wearable support element. Referring to Figure 8, the garment-like or wearable support element 10 is formed of a band, a strip or the like, which may be removably fastened around the body under examination or a part of such body under examination. Such strip, band or the like has two mutual connection ends, with means 11 for removably fixing and fastening the band or strip around a part of the body under examination, such as Velcro means and/or button-like means and/or other means such as laces or pairs of straps that can be connected together by interlocking end buckles, having strap pulling means. The garment-like or wearable support element 10 may advantageously be at least partly or wholly made of an elastically extensible material.

The antenna element and/or the conductors 9 that form it are coupled to the garment-like or wearable support element 10 by receiving means, such as loops, waistbands, pockets or combinations thereof, or by permanent or removable fastener means, as shown in Figures 8 to 11 and designated by numeral 12. The above receiving means are not shown in detail for their being readily understandable without being illustrated.

The removable fastener means 12 may advantageously consist of Velcro strap elements. For a single wearable element 10 to be used for several different coils or receiving means having different structures or shapes and particularly formed of conductors that extend in different predetermined paths, the garment-like or wearable element has a certain number of fastener elements 12 arranged on its surface in a predetermined pattern or order The fastener elements 12, in the form of connecting strap elements, cooperate with complementary elements of such connecting strap, that are attached to the rigid or flexible conductors and/or to structural elements of the coils or receiving means in general.

Alternatively or additionally, the garment-like or wearable support element has loops and/or waistbands for receiving rigid or flexible conductors and/or segments of rigid or flexible conductors that form the antenna element or parts of the receiving means or receiving coils.

According to a further variant embodiment, the removable fastener means for rigid or flexible conductors and/or segments of rigid or flexible conductors and/or loops and/or the waistbands for receiving rigid or flexible conductors and/or segments of rigid or flexible conductors are formed and/or fixed to the garment-like and/or wearable support element and/or to the rigid or flexible conductors and/or segments of rigid or flexible conductors by means such as elastically extensible and/or deformable bridges of material and/or fabric.

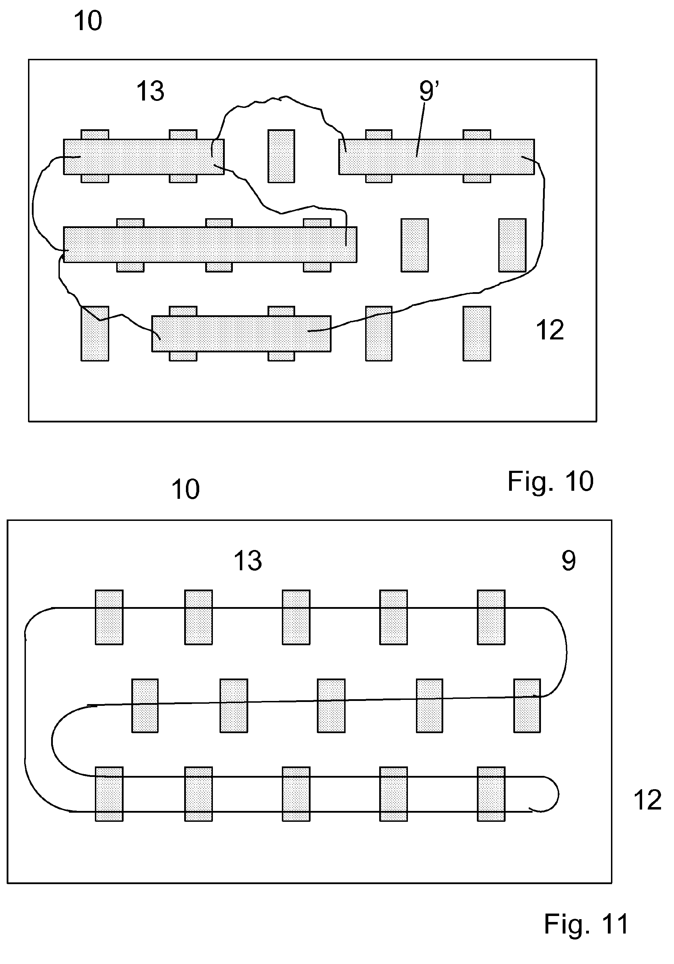

As mentioned above and shown in Figures 10 and 11, the receiving means, i.e. the antenna element, may be formed of a one-piece construction element designated by numeral 9 in Figure 11 and comprising rigid conductors which extend in a predetermined rigid three-dimensional path or a single construction element composed of one or more elastically or inelastically flexible and/or extensible conductors.

In order that the patient may be allowed to assume several different postures using a single receiving means, in addition to the use of elastically or inelastically flexible conductors, the invention provides, as shown in Figure 10, an antenna element, i.e. a receiving coil, composed of one or more segments 9' of rigid and/or elastically or inelastically flexible and/or extensible conductors, which may be connected together by removable rigid and/or elastically or inelastically flexible and/or extensible electric connection bridges.

The elastically or inelastically flexible and/or extensible conductors 9 or said one or more segments 9' of rigid and/or elastically or inelastically flexible and/or extensible conductors, that may be connected together by removable electric connection bridges 13, which in turn can be rigid and/or elastically or inelastically flexible and/or extensible, have means for removable attachment to cooperating removable fastener elements 12 on the garment-like or wearable support element 10, which removable fastener means 12 and which cooperating attachment means are respectively arranged along the corresponding elastically or inelastically flexible and/or extensible conductors 9 or the one or more segments 9' of rigid and/or elastically or inelastically flexible and/or extensible conductors and along the surface of the garment-like and/or wearable support element 10. The above is provided in such a manner that, as each removable attachment means is coupled with each corresponding removable fastener element, the flexible conductors 9 and/or the segments 9' of rigid and/or flexible conductors are automatically shaped and/or positioned relative to each other in a predetermined three-dimensional path pattern of said conductors to obtain the shape of the antenna or the receiving coil.

Therefore, the receiving coil is formed of a plurality of segments 9' of rigid or flexible conductors that may be removably attached to the garment-like and/or wearable support element 10, and may be electrically connected together by removable electric connection bridges 13, a set of such segments 9' of conductors being provided, which comprises pieces of different lengths and/or shapes for modular construction of conductors of different lengths and shapes, such as rectilinear segments of different lengths and/or curved segments having different lengths and/or different curvatures.

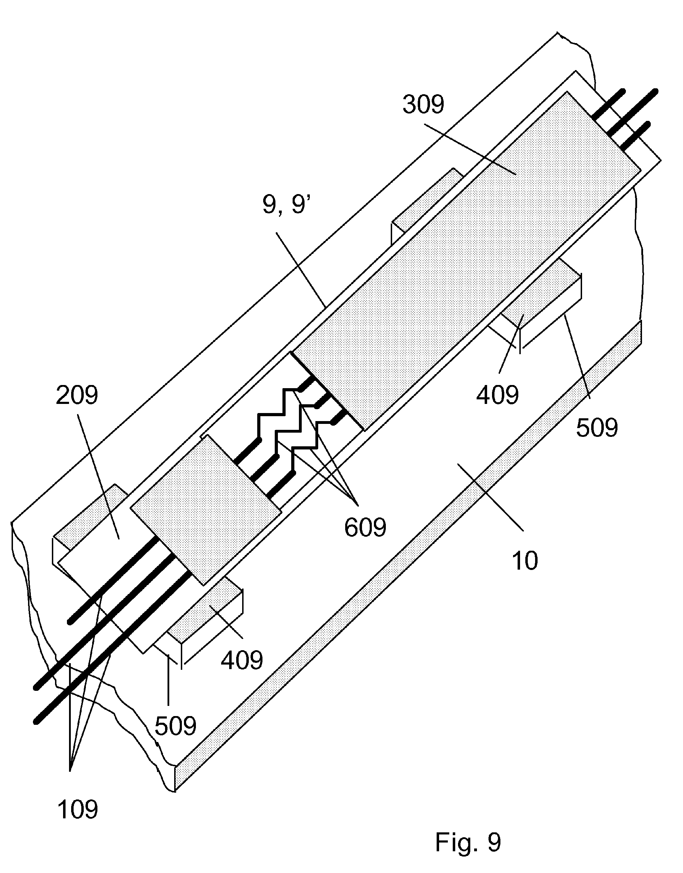

Figure 9 shows an enlarged detail of a possible embodiment of an elastically or inelastically flexible and/or extensible conductor 9, or a segment 9' of rigid and/or elastically or inelastically flexible and/or extensible conductor. In this case, one or more conductors consisting of a wire, a strap or a track of electrically conductive material 109 are held in a channel between two straps of flexible and/or extensible material, designated by numerals 209 and 309, which are fixed together, for instance glued and/or sewn and/or welded along their two lateral longitudinal edges. The strap 209 on the wearable garment side 10 consists of one of the two strap parts of a Velcro removable fastening device or has such strap part of the Velcro coupling device attached thereon all along its length or at parts thereof. These strap parts 409 engage with corresponding and complementary strap parts 509 which are fixed in coincident positions on the surface of the garment-like element 10.

According to a further feature allowing to use elastically and/or inelastically conductor segments 9' or conductors 9, the wires, straps or tracks 109 of conductive material have high-density portions arranged along part or all of their length, such as zigzag, wavy and/or spiral sections, or the like. These sections, designated by numeral 609 in Figure 9, include excess lengths of wire or the like. The provision that at least in such areas or all along their length, the wires 109 are held in an elastically extensible and/or deformable sheath that is formed, for instance, by the two straps 209 and 309, allows the conductor or a conductor segment 9, 9' to form a flexible and elastically extensible receiving coil, which can fit a variety of patient postures.

In a further variant, the attachment means 13, like the Velcro elements, are attached to the wearable element 10 using bridges of elastically deformable and/or extensible material.

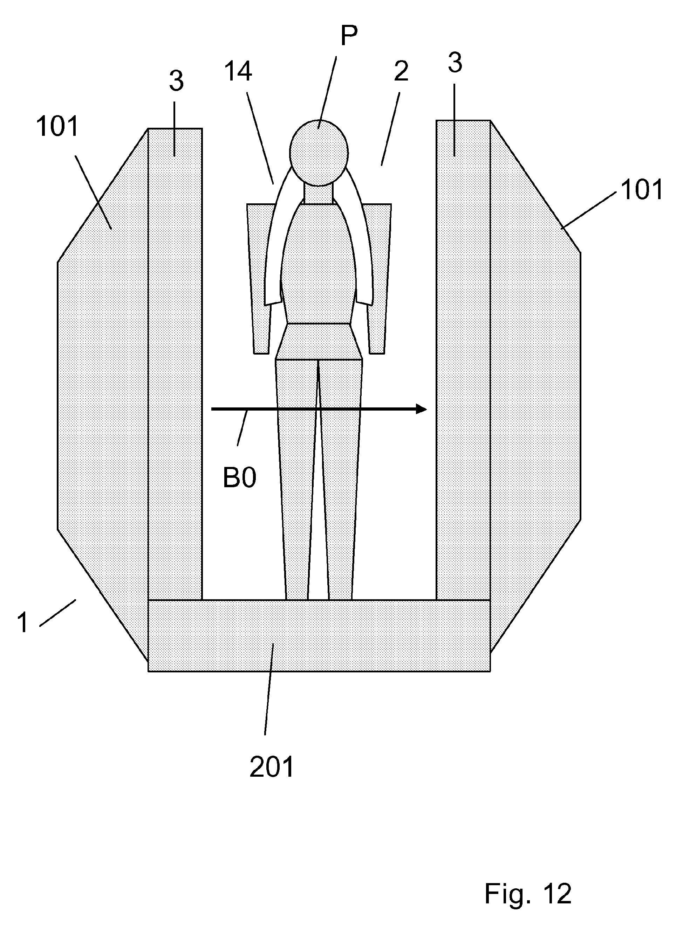

According to another feature of the invention, to be used separately or in addition to those described above, the apparatus is provided in combination with means for adjusting gravity stresses on the body under examination, a non limiting example thereof being shown in Figure 12.

Particularly referring to the example of Figure 12, these means, designated by numeral 14, consist of a garment-like and/or wearable element containing one or more ballast elements. The ballast elements have a predetermined weight and may be permanently and/or removably connected to the garment element and/or replaced with other ballast elements of different weight.

Advantageously, the garment-like and/or wearable element is the same element as the one that is used for supporting the antenna or coil element, suitably having connections, pockets or the like for holding one or more ballast elements.

The method of use of the inventive apparatus is apparent from the above description. In the MRI imaging method with the apparatus of the invention, the patient
- accesses the imaging cavity, or the patient receiving space 2, by simply walking therein,
- autonomously assumes a position, or can autonomously assume a position as instructed by the operating personnel;
- is submitted to an imaging process.

For a better and more effective patient placement, shapes and/or positioning marks may be drawn, possibly in different colors, on the walking surface of the patient receiving space 2, here corresponding to the base 201 and/or on one or more of the walls of the magnet structure which delimit such patient receiving space 2, here corresponding to the vertical walls 101 and/or to the magnetic field generating means 3, such shapes being provided for use by the operating personnel as patient positioning examples. Therefore, in this case, the patient may be asked to place his/her feet in the feet positioning shapes on the floor of the space 2, which have a predetermined color, and to assume a posture as indicated by a body shape, a silhouette or the like, having a predetermined color or defined by an outline of a predetermined color on one or both vertical walls 101 or on the means 3.

When the receiving means are integrated in the magnet structure, the patient may avoid to wear or have one or more receiving coils associated thereto.

In the previous case, especially when the magnet structure has no receiving coils integrated therein, before entering the patient receiving space 2 and/or possibly simply before assuming the posture required for the examination and before being submitted to imaging, the patient has to wear one or more receiving coils as described above and/or have a possibly different receiving coil associated thereto, which coil is supported by support means associated to the magnet structure and may be displaceable to the operating position, in which it is coupled with the patient body, and to an idle position, in which it is far from the patient body.

When an examination requires means for assisting the patient in assuming a certain posture, then the examination includes the steps of:
- introducing a means for positioning and/or bearing and/or retaining and/or supporting a patient or for assisting placement thereof in the imaging space, in a predetermined position for the type of examination to be performed with reference to the anatomic region to be imaged;
- having the patient autonomously access the imaging space and autonomously sit or lie on such means for positioning and/or bearing and/or retaining and/or supporting the patient or for assisting placement thereof;
- so that the patient assumes the position or posture imposed by such means for positioning and/or bearing and/or retaining and/or supporting the patient or for assisting placement thereof;
- carrying out the MR imaging process;
- having the patient autonomously leave such means for positioning and/or bearing and/or retaining and/or supporting the patient or for assisting placement thereof;
- having the patient leave the imaging space by autonomous walking.

Alternatively, when the means for positioning and/or bearing and/or retaining and/or supporting the patient or for assisting placement thereof have a carriage-mounted base and when such means for positioning and/or bearing and/or retaining and/or supporting the patient or for assisting placement thereof can access the patient receiving space 2 through the access opening, then, as an alternative to the above, the patient sits or lies on said means for positioning and/or bearing and/or retaining and/or supporting the patient or for assisting placement thereof when such means are outside the patient receiving space as delimited by the magnet structure and then accesses such patient receiving space on such means for positioning and/or bearing and/or retaining and/or supporting the patient or for assisting placement thereof, driven or pushed by external means, which may be manual and patient-driven or motor-driven under the control of the patient or third parties.

As patient placement means a platform is provided on which the patient is asked to climb, which platform has an access side with one or more steps, the patient being positioned with both legs on a step or on the top of the platform or with one leg resting on the first or on one of the next steps, and the other leg resting on the base of the imaging space and/or on the top of the base.

Alternatively or additionally, as placement means, a chair element is provided, having a seating surface, a backrest and possibly a footrest, one or more of such seating, backrest and/or footrest surfaces being adjustable in position and/or tilt and/or orientation.

Once the patient is in the sitting position, either inside the patient receiving space or outside it, as provided by the above alternative methods, the seating surface and/or the backrest and/or the footrest may be adjusted in position and/or tilt or orientation.

Then, with the patient sitting and positioned within the patient receiving space, MR imaging is performed.

The position and/or tilt or orientation of the seating surface and/or the backrest and/or the footrest may be changed a first time and/or one or more additional times, and MR imaging may be repeated each time.

Once imaging is completed, the patient is either asked to stand up while still in the patient receiving space and to leave the space by autonomous walking, or carried out of the space on the chair, using the latter as a means of transport.

When using patient bearing and/or retaining means that may be set in various positions within the imaging space, the imaging method includes the steps of:
- positioning one or more of such retaining elements relative to the magnet and to a patient position, so that the patient assumes a predetermined position when he/she rests on such retaining means;
- having the patient access the imaging space by autonomous walking and autonomously assume the above mentioned position;
- carrying out the MR imaging process;
- possibly adjusting the position of one or more retaining elements or provide one or more further retaining elements in one or more further different positions within the imaging space and repeating the MR imaging process each time;
- having the patient stand up and leave the imaging space by autonomous walking.

Imaging may also be performed by one or more successive imaging steps, the patient being asked, before at least one of such steps or some of such steps, to wear a garment or a wearable and removably fastenable element, which acts as a ballast element 14.

In one embodiment, a sequence of successive MR imaging operations is performed, the weight of the ballast element being increased or decreased before each imaging operation, from a predetermined initial value and/or according to predetermined variation functions.

According to yet another feature of the invention, the patient performs a predetermined movement during MR imaging, such as a walking and/or climbing movement, the base being provided in the form of a tilting treadmill and/or a step-up or step-down movement and/or a sitting movement or a passage from the sitting position to the standing position, or a forward bending movement or a backward and/or sideward arching movement, while a time-dependent sequence of MR imaging operations on at least one or more anatomic regions is performed during such movements.

## Claims

1. A MRI apparatus comprising a magnet structure which delimits a cavity in or through which a body under examination or a part thereof is received, and which includes means for generating a magnetic field in said cavity, as well as means for causing the body under examination or the part thereof to emit nuclear magnetic resonance signals and means for receiving said nuclear magnetic resonance signals
**CHARACTERIZED IN THAT**
said magnet structure delimits a cavity whose shape provides at least one access opening through which patients can access said cavity by simply walking therein.

2. An apparatus as claimed in claim 1, **characterized in that**
the magnet structure has at least two opposite magnetic field generating pole pieces which form or are supported by two opposite vertical walls which are spaced to such an extent as to form a patient imaging space and at least one access opening at least at one end side;
a base that acts as a connection wall between the two opposite walls that form or support the pole pieces of the magnet structure, which base has one, two or more steps on the opening side, for access to the imaging space and/or at least two parallel climbing ramps at the at least one access opening, at least along parallel strips disposed at the same distance from each other as the wheels of a wheelchair or the like, or one climbing ramp whose width corresponds to the total width of the base.

3. An apparatus as claimed in claim 1 or 2, **characterized in that** it has a balustrade and/or lateral gripping and retainer means may be further at least in the area of the steps and/or ramps and at least on one side or on both sides thereof.

4. An apparatus as claimed in one or more of the preceding claims, **characterized in that** it includes means for positioning and/or bearing and/or retaining and/or supporting a patient or for assisting placement thereof, which are mountable and removable in or out of the imaging space separately or in combination.

5. An apparatus as claimed in claim 4, **characterized in that** said positioning means consist of one or more platforms having different heights and an access side composed of one or more steps, which platforms may be removably mounted in several different positions to the base of the magnet structure within the imaging space.

6. An apparatus as claimed in claim 5, **characterized in that** there is provided a modular platform composed of multiple platform elements, each having a predetermined unit height, which platform elements may be mounted in superimposed arrangements for making platforms of various heights and/or in laterally staggered positions for forming a stepped access side.

7. An apparatus as claimed in one or more of claims 4 to 6, **characterized in that** it comprises several gripping handles or other gripping means, in several different positions with respect to the walls that form and/or support the pole pieces and/or with respect to the base and/or with respect to the platform, which gripping handles or other gripping means are at least partly permanently mounted in various fixed positions, at least some of said handles or said gripping means being mounted or mountable removably and/or adjustably in terms of position and orientation in various positions with respect to the extension of the walls that support or form the pole pieces and/or the base and/or the platform/s.

8. An apparatus as claimed in claim 7, **characterized in that** the gripping handles and/or the other gripping means are arranged to be removably and/or adjustably mountable in position to support elements such as rods, posts and/or beams, which support elements may be removably fixed in one or more predetermined positions to the base and/or the walls or the pole pieces of the magnet structure and/or the platform/s or the modular platform elements.

9. An apparatus as claimed in claim 7 or 8, **characterized in that** the elements for supporting the gripping means and/or the handles are formed by multiple elements, that can be removably coupled together and/or extended or shortened or anyway have an adjustable size.

10. An apparatus as claimed in one or more of the preceding claims 4 to 9, **characterized in that** it has at least one handle, preferably a pair of laterally aligned or misaligned handles, which are spaced in such a position as to provide support for backward arching of the standing patient body.

11. An apparatus as claimed in one or more of the preceding claims 4 to 9, **characterized in that** it has at least one handle, preferably a pair of laterally aligned or misaligned handles, which are spaced in such a position as to provide support for forward bending of the standing patient body.

12. An apparatus as claimed in one or more of the preceding claims 4 to 11, **characterized in that** it has at least one seat element that may be removably mounted or positioned in different positions within the imaging space and on the base thereof.

13. An apparatus as claimed in one claim 12, **characterized in that** the seat is provided in combination and is mountable with one or more of the patient positioning and/or retaining and/or gripping and/or supporting elements as claimed in claims 4 to 11.

14. An apparatus as claimed in one or more of the preceding claims 4 to 6, **characterized in that** the means for positioning and/or bearing and/or retaining and/or supporting the patient or for assisting placement thereof act at least partly as guides or cases for receiving several different devices or means, such as the resonance signal receiving means and the cables for connecting the outputs of such means to an electronic processor unit of the apparatus, associated to the magnet structure and located outside the latter.

15. A MRI method using a MRI apparatus comprising a magnet structure which delimits a cavity in or through which a body under examination or a part thereof is received, and which includes means for generating a magnetic field in said cavity, as well as means for causing the body under examination or the part thereof to emit nuclear magnetic resonance signals and means for receiving said nuclear magnetic resonance signals,
said cavity has a shape that provides at least one access opening through which patients can access said cavity by simply walking therein, and said method comprises the steps of:
- having the patient access the imaging cavity by autonomous walking,
- positioning the patient by having him/her move autonomously as instructed by the operating personnel;
- performing MR imaging.
and **characterized in that** the patient positioning steps include the steps of:
- introducing a means for positioning and/or bearing and/or retaining and/or supporting a patient or for assisting placement thereof in the imaging space, in a predetermined position for the type of examination to be performed with reference to the anatomic region to be imaged;
- having the patient autonomously access the imaging space and autonomously sit or lie on such means for positioning and/or bearing and/or retaining and/or supporting the patient or for assisting placement thereof;
- so that the patient assumes the position or posture imposed by such means for positioning and/or bearing and/or retaining and/or supporting the patient or for assisting placement thereof;
- carrying out the MR imaging process;
- having the patient autonomously leave such means for positioning and/or bearing and/or retaining and/or supporting the patient or for assisting placement thereof;
- having the patient leave the imaging space by autonomous walking.

16. A method as claimed in claim 45 or 46, **characterized in that**, as patient placement means, a platform is provided on which the patient is asked to climb, which platform has an access side with one or more steps, the patient being positioned with both legs on a step or on top of the platform or with one leg resting on the first or on one of the next steps, and the other leg resting on the base of the imaging space and/or on top of the base.
